# EUROPÄISCHE PATENTANMELDUNG

(11) **EP 2 286 755 A1**
(43) Veröffentlichungstag der Anmeldung: **23.02.2011**
(21) Anmeldenummer: 10187013.7
(22) Anmeldetag: 06.07.2006
(51) Int. Cl.: A61B 18/22

(54) **Vorrichtung für die Laserchirurgie**

(30) Priorität: 26.06.2006 US 426415
(62) Teilanmeldung aus: 06754687.9
(71) Anmelder: Rolle + Rolle GmbH & Co KG, 5020 Salzburg (AT)
(72) Erfinder: Koeth, Johannes Bernhard, 97218, Gerbrunn (DE); Rolle, Ingeborg, 82291, Mammendorf (DE)
(74) Vertreter: Grünecker, Kinkeldey, Stockmair & Schwanhäusser Anwaltssozietät

(57) **Zusammenfassung**

Die Erfindung betrifft eine Vorrichtung für die Laserchirurgie mit einem Laser und Mitteln zum Einkoppeln von Laserlicht des Lasers in eine Lichtleitfaser wobei der Laser ein Festkörperlaser ist, der Laserlicht ausstrahlen kann, das bei der Maximalintensität eine Wellenlänge im Bereich zwischen 1100 und 1400 nm hat, wobei der Laser mindestens eine Ausgangsleistung von 25 W hat.

## Beschreibung

Die Erfindung betrifft eine Vorrichtung für die Laserchirurgie mit einem Laser und Mitteln zum Einkoppeln von Laserlicht des Lasers in eine Lichtleitfaser.

Derartige Vorrichtungen für die Laserchirurgie sind beispielsweise aus der DE 91 162 16 U bekannt. Auch die DE 44 08 746 C2 zeigt einen Laserkatheter zur Bypasschirurgie. Hier werden zum Beispiel Nd: YAG-Laser erwähnt. Diese Laser weisen eine Laserlinie bei 1064 nm auf.

Aufgabe der vorliegenden Erfindung ist es, eine Vorrichtung für die Laserchirurgie zu verbessern. Diese Aufgabe wird gelöst durch eine Vorrichtung nach Anspruch 1.

Für die Laserchirurgie von Geweben im Allgemeinen, insbesondere aber für die Chirurgie von Lungengewebe, hat sich herausgestellt, dass eine Wellenlänge zwischen 1100 nm und 1400 nm vorteilhaft ist. In diesem Wellenlängenbereich stellt sich eine Absorption der Laserstrahlung in wasserhaltigem Gewebe also auch Lungengewebe ein, die es erlaubt zügig das Gewebe zu durchtrennen oder zu schneiden und gleichzeitig eine ausgedehnte Koagulation an stark durchblutetem Gewebe zu erzeugen. Beim Lungengewebe wird ebenfalls gleichzeitig ein weiterer sehr wichtiger Effekt nämlich die Verschweißung von Luftfistein erreicht. In diesem Wellenlängenbereich sind zwar Lasersysteme verfügbar, jedoch sind diese in der Regel sehr unhandlich, da beispielsweise Gaslaser in der Regel eine aufwendige Wasserkühlung benötigen, sodass ein Gerät dieser Art sehr schwer ist oder die verfügbaren Ausgangsleistungen zu gering sind.

Für die Laserchirurgie im oben genannten Wellenlängenbereich ist ein Laser mit einer Ausgangsleistung von mindestens 25 W wünschenswert.

Auf Grund kürzlicher materialtechnischer Entwicklungen ist es möglich, Festkörperlaser wie etwa Halbleiterlaser und/oder Faserlaser mit derartigen Ausgangsleistungen, wie sie für die Laserchirurgie, insbesondere für die Lungenlaserchirurgie, benötigt werden, zur Verfügung zu stellen und dabei eine Wellenlänge im Bereich von 1100 bis 1400 nm zu erzeugen.

Besonders bevorzugt ist ein Wellenlängenbereich oberhalb von 1150, 1175, 1200, 1250 1275, 1300, 1308 oder 1310 nm. In diesem Wellenlängenbereich zeigt die Absorption von Wasser eine nicht allzu große Abhängigkeit von der Wellenlänge, sodass bestimmte Wellenlängenschwankungen in diesem Bereich nicht automatisch zu einem anderen chirurgischen Verhalten der Laserstrahlung führen. Andererseits sollte die Laserstrahlung auch unterhalb von einer Wellenlänge von 1350, 1330, 1325, 1300, 1275, 1250, 1225 oder 1200 nm liegen, da ansonsten der Plateaubereich verlassen wird. Auf diesem Plateaubereich stellt sich eine relativ günstige Absorption und ein vorteilhaftes Verhältnis von Absorption zu Streuung in Geweben mit relativ hohem Wassergehalt und somit auch in Lungengewebe ein.

Vorteilhaft sind weiterhin Laser mit einem Laserlicht, das eine gewisse spektrale Halbwertsbreite aufweist. Hier wird die spektrale Breite des Spektrums bei der Hälfte der Maximalintensität bestimmt. Die Halbwertsbreite beträgt z. B. mindestens 2, 3, 4, 5, 7, 10, 12 oder 15 nm. Die Obergrenze für die Halbwertsbreite kann z. B. 2, 3, 4, 5, 7, 10, 12, 15, 20, 25, 30 oder 40 nm sein. Durch die Abdeckung eines größeren Spektralbereichs wird die Vorrichtung gegen geringe Wellenlängenschwankungen unempfindlicher, sodass ein konstantes Chirurgieverhalten des Laserlichts während einer Operation gewährleistet wird. Andererseits soll das Spektrum auch nicht zu breit sein, damit keine Spektralanteile mit einer zu hohen Absorption in dem Gewebe vorliegen.

Die Ausgangsleistung des Lasers ist bevorzugterweise größer als 25 W. Hier ist beispielsweise eine Leistung von mindestens 80 W bevorzugt.

Der Laser kann sowohl ein Dauerstrich als auch ein gepulster Laser sein.

Vorteilhaft ist weiterhin ein Laser, der mehrere Laserelemente umfasst. Dies können mindestens oder genau 2, 5, 10, 15, 19, 20, 25, 30, 35, 38, 40, 50, 60, 70, 80, 90, 100, 150 Laserelemente sein. Die einzelnen Laserelemente können so mit einer geringeren Leistung als mit der gewünschten Gesamtausgangsleistung betrieben werden, was die Lebensdauer dieser Laserelemente erhöht bzw. größere Ausgangsleistungen erst ermöglicht.

Weiterhin sind hier vorteilhafterweise Mittel vorgesehen, mit denen die Laserstrahlen dieser einzelnen Laserelemente in eine gemeinsame Lichtleitfaser eingekoppelt werden können. An sich ist es auch denkbar, für jedes Laserelement eine einzelne Lichtleitfaser vorzusehen. Diese einzelnen Fasern können dann gebündelt werden und das Licht derselben erst am Ende der Lichtleitfaser mit einer Optik zusammengeführt werden. Derartige Mittel zum Einkoppeln von Laserlicht können eine oder mehrere Linsen und/oder Spiegel umfassen, wobei hier auch zylindrische Linsen/Linsenarrays und/oder Spiegel vorgesehen sein können.

Die einzelnen Laserelemente und/oder Laser können Halbleiterlaser und/oder Faserlaser sein oder umfassen. So können beispielsweise einzelne Faserlaser gebündelt angeordnet werden, um das Licht in eine gemeinsame Ausgangslichtleitfaser zu geben. Hier können aber auch ein oder mehrere Faserkoppler vorgesehen sein, mit denen das Licht aus zwei oder mehr Fasern in eine einzelne Faser eingekoppelt wird.

Auch mit Halbleiterlasern kann im gewünschten Wellenlängenbereich die benötigte Ausgangsleistung erreicht werden. Hierzu wird in der Regel über eine Strahloptik das Licht von mehreren Laserelementen in eine Lichtleitfaser eingekoppelt.

Die Halbleiterlaser können entweder elektrisch oder optisch gepumpt werden. Ein optisches Pumpen kann z.B. mit anderen Halbleiterlasern mit einer Wellenlänge kürzer als 1100 nm erfolgen. Die Faserlaser werden in der Regel optisch gepumpt wie etwa mit Halbleiterlaserdioden, wie sie in diesem Dokument beschrieben werden. In der Regel werden die Pumplaser eine Wellenlänge von unterhalb 1100 nm haben, jedoch zumindest unterhalb der Wellenlänge des Faserlasers.

Vorteilhafterweise ist der Laser ein Quantenpunktlaser bzw. ein Quantenfilmtaser. Mit derartigen Laserelementen ist es mit einer vertretbaren Anzahl von Laserelementen möglich in dem gewünschten Wellenlängenbereich die benötigte Ausgangsleistung zur Verfügung zu stellen. Derartige Quantenpunktlaser können auf dem GaAs-AlGaAs-Materialsystem beruhen. Die Quantenpunkte des Quantenpunktlasers können hierbei in Quantenfilmen (auch Quantentröge genannt) vorgesehen sein, sodass das Confinement der Ladungsträger im Bereich der Quantenpunkte durch das Confinement der Ladungsträger in dem Quantenfilm verbessert wird.

Quantenpunkte können jedoch auch ohne Quantenfilme bzw. außerhalb von eventuellen Quantenfilmen vorgesehen sein.

Quantenpunkte können GatnAs, GalnAsN und/oder GainSb umfassen oder daraus bestehen. Die Quantenpunkte haben eine Bandlücke, die geringer ist als diejenige des umgebenden Materials. Dadurch werden die Ladungsträger auf Energieniveaus, die u. a. durch die Größe des Quantenpunkts vorgegeben werden, gebracht, sodass hier auch Laserwellenlängen möglich werden, die nicht der Bandlücke des Quantenpunktmateriais entsprechen.

Der Wellenleiter des Halbleiterlasers bestimmt die Lichtführung innerhalb des Halbleitermaterials. Hier wird für den Laser für die Vorrichtung für die Laserchirurgie ein relativ breiter Wellenleiter bevorzugt, da das Licht sich somit über einen großen Bereich in dem Halbleiterlasermaterial erstreckt und die Inhomogenitäten durch die Ausbildung von Quantenpunkten, Quantenfilmen oder sonstigen Grenzflächen hohe Leistungen nicht behindern, beispielsweise durch Streuung an Grenzflächen oder Grenzflächendetekten oder an den Quantenpunkten.

Der Wellenleiter hat eine Breite von mindestens 200. 250, 300, 400, 500 oder 600 nm. Der Wellenleiter wird durch eine Brechungsindexdifferenz zwischen dem Wellenleiterinnerem und dem Weltenleiteräußeren definiert. Der Wellenleiter ist bevorzugterweise so ausgebildet, dass sich nur eine transversale Lichtmode einstellt, Es können aber auch zwei oder drei transversale Moden gegeben sein, da so höhere Leistungen möglich werden, ohne jedoch ein wohldefiniertes Stahlprofil zu verlieren.

Der Wellenleiter kann GaₓIn₍₁₋ₓ₎AsᵤPᵥN_{w}Sb_{(1-u-v-w)} umfassen oder daraus bestehen, wobei x, u, v und w die Werte 0 bis 1 und alle Zwischenwerte oder Werte aus allen möglichen Zwischenintervallen annehmen kann und u+v+w kleiner oder gleich 1 ist.

Der Halbleiterlaser kann auch ein Quantenfilmlaser sein, mit einem Quantenfilm der GaᵤIn₁₋ᵤ, AsₓN_{y}P_{1-x-y} umfasst oder daraus besteht, wobei u, x und y die Werte 0 bis 1 oder alle Zwischenwerte oder Werte aus allen möglichen Zwischenintervallen annehmen kann solange x+y≤1 ist. Mit derartigen Materialien sind gewünschte Ausgangsleistungen in dem vorgegebenen Wellenlängenbereich möglich. Dies erfolgt unter Zusammenfassung von einer relativ großen Anzahl von Laserelementen, damit die benötigte insgesamte Ausgangsleistung des Lasers erreichbar erscheint.

Auch Festkörperscheibenlaser sind für die Erzeugung von Hochleistungslasem in dem benötigten Wellenlängenbereich vorteilhaft möglich. Bei diesen Lasern hat ein Kristall eine Scheibenform und ist auf einer Seite verspiegelt. Das Laserlicht tritt auf der gegenüberliegenden Seite aus. Solche Scheiben können sehr gut gekühlt werden, so dass hohe Leistungen möglich werden.

In jedem Fall sind zylindrische Linsen und/oder Linsenarrays und/oder Spiegel für die Einkopplung des Lichts von mehreren Laserelementen vorteilhaft.

Die Vorrichtung weist eine Kopplung auf, an die lösbar Lichtleitfasern angeschlossen werden können. Während einer Operation werden die Lichtleitfasern leicht verschmutzt, sodass diese auswechselbar sein sollten.

Die Lichtleitfaser weist vorteilhafterweise ein Griffteil auf, da dies die Manipulation der Lichtleitfaser vereinfacht. In dem Griffteil kann auch eine Optik vorgesehen sein, mit der das aus der Lichtleitfaser austretende Licht auf einen Fokus fokussiert wird. Zwingend notwendig ist dies jedoch nicht, da ein Bestrahlungsbereich von mehreren Millimetern Durchmesser auch ohne Optik erreichbar ist.

Die Optik ist vorzugsweise ebenfalls auswechselbar. Zum Einen wird die Optik auch leicht verschmutzt, zum Anderen können mit verschiedenen Optiken verschiedene Arbeitsabstände und verschiedene Fokusgrößen erreicht werden.

Die Vorrichtung weist weiterhin eine Luftkühlung zum Kühlen des Lasers bzw. der Vorrichtung auf. Eine Wasserkühlung ist hier nicht vorgesehen, da die Kühlluftkühlung bei einem Halbleiterlaser ausreichen sollte. Eine Wasserkühlung wird jedoch auch nicht ausgeschlossen.

Weiterhin kann die Vorrichtung eine Temperaturregeleinrichtung aufweisen, mit der die Temperatur des Halbleiterlasers eingestellt werden kann. Dies kann beispielsweise ein Peltierelement umfassen. Dies dient zum Einen dazu die Abwärme abzuführen. Andererseits kann mit der Temperatur auch die Wellenlänge des emittierten Lichts eingestellt werden. Das Peltierelement kann wasser- und/oder luftgekühlt sein. Auch ist lediglich eine Wasserkühlung ohne Peltierelement möglich. Die Wasserkühlung kann hierbei aber auch recht klein ausfallen.

Bevorzugte Ausführungsformen der Erfindung sollen anhand der beiliegenden Figuren erläutert werden. Dabei zeigt:
- Figur 1: eine Vorrichtung für die Laserchirurgie;
- Figur 2: eine schematische Schnittansicht des distalen Endes der Lichtleitfaser;
- Figur 3: die Anordnung von dem Halbleiterlaser, einer Einkoppeloptik und einem Fase- rende in schematischer dreidimensionaler Ansicht;
- Figur 4: schematische dreidimensionale Ansichten einer Laseranordnung;
- Figur 5: eine schematische Ansicht des Aufbaus eines Halbteiterquantenpunktlasers;
- Figur 6: ein beispielhaftes Spektrum des Lasers.

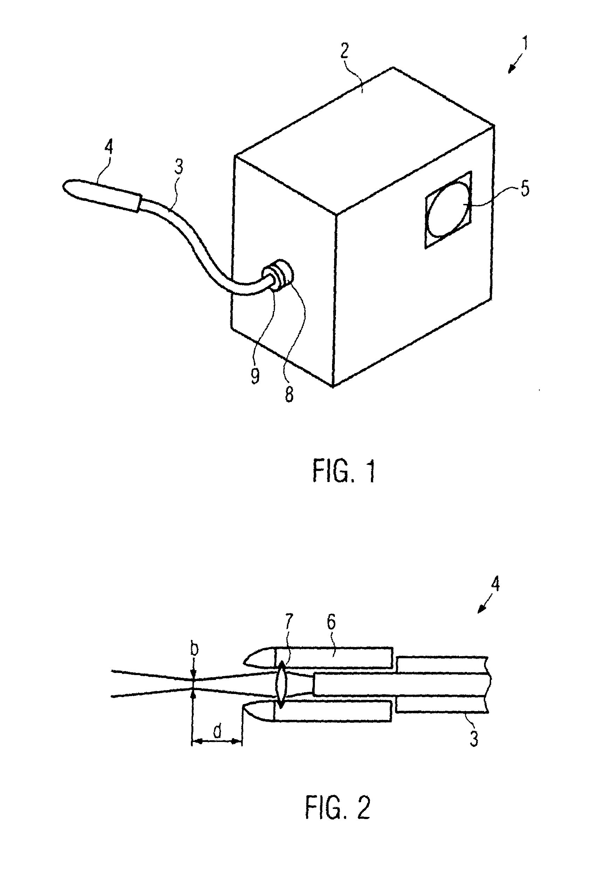

In Figur 1 ist eine Vorrichtung 1 für die Laserchirurgie gezeigt. Die Vorrichtung umfasst ein Gehäuse 2 mit einer Kopplung 8, in die eine Lichtleitfaser 3 mit einem Stecker 9 eingekoppelt werden kann. Die lichtleitfaser 3 hat ein Ende mit einem Griffteil 4. Das Gehäuse 2 verfügt weiterhin über einen Lüfter 5 für die Luftkühlung.

Das Ende der Lichtleitfaser 3 mit dem Griffteil 4 ist in Figur 2 schematisch in einer Schnittansicht dargestellt. Die Lichtleitfaser 3 endet in einem abnehmbaren Optikhalter 6, in dem eine Optik 7, hierzu symbolisch durch eine einzelne Linse 7 dargestellt, angeordnet ist. Das aus der Lichtleitfaser 3 austretende Licht wird durch diese Optik gebündelt, sodass sich in einem Arbeitsabstand d ein Fokus mit einer Halbwertsbreite b ausbildet. Die Lichtleitfaser 3 hat einen lichtleitenden Faserkem sowie eine Ummantelung. Der bevorzugte Arbeitsabstand d beträgt einige Zentimeter. Besonders bevorzugt ist ein Arbeitsabstand von 1 bis 5 cm, wie etwa 1,5 cm (± 0,5 cm) oder 2,5 cm (t 1,0 cm) oder 3,5 cm (± 1,0 cm) oder 4,5 cm (± 0,5 cm). Die Halbwertsbreite b im Fokusbereich beträgt einige Millimeter. Der Strahldurchmesser im Fokus kann beispielsweise 0,5 mm sein.

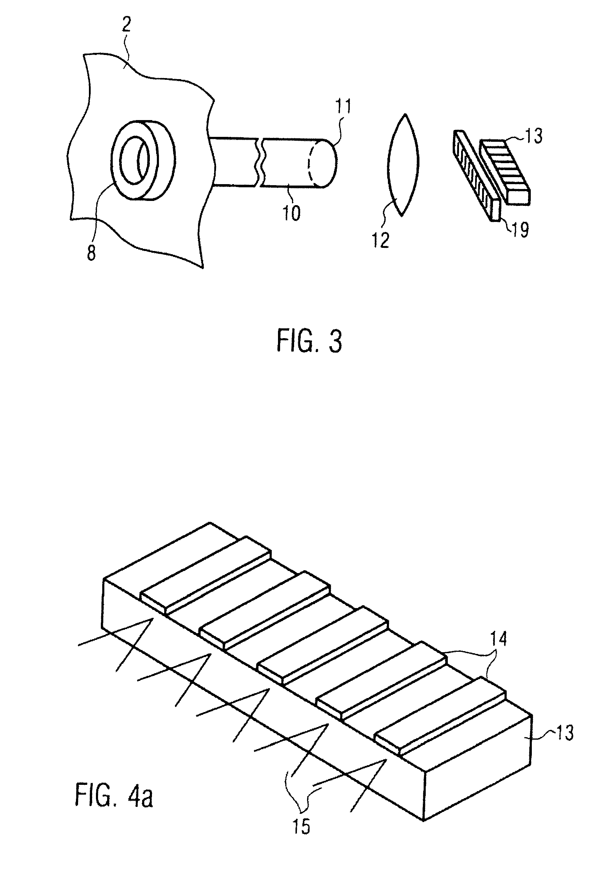

In Figur 3 ist ein Teil des Gehäuses 2 mit der Kopplung 8 dargestellt. Hier ist schematisch ein Faserende 11 einer internen Lichtleitfaser 10 dargestellt, in die das Licht des Lasers 13 eingekoppelt wird. Der Laser 13 ist hier als Laserbarren ausgeführt. Zwischen dem Laserbarren 13 und dem Ende 11 der Lichtleitfaser 10 ist eine Einkoppeloptik 12 vorgesehen, mit der das divergent aus dem Barren 13 austretende Licht auf das Lichtleitfaserende 11 gebündelt wird. Die Einkoppeloptik kann eine zylindrische Linse oder einen zylindrischen Spiegel umfassen. Bevorzugt ist beispielsweise auch ein Zylinderlinsenarray 19, wobei jedem Laserelement bevorzugt ein Zylinderlinsenelement des Zylinderlinsenarrays zugeordnet ist.

Der Barren 13 ist in Figur 4a schematisch dargestellt. In ihm sind verschiedene Halbleiterlaserelemente 14 angeordnet, wobei aus jedem Element 14 ein Lichtlaserstrahl 15, der sehr divergent sein kann, austritt.

In dem Barren 13 in Figur 4a sind Laserelemente 14 nebeneinander angeordnet. Ein solcher Laserbarren kann bis zu 10, 15, 20, 25, 30, 35, 40, 45 oder 50 Laserelemente 14 umfassen.

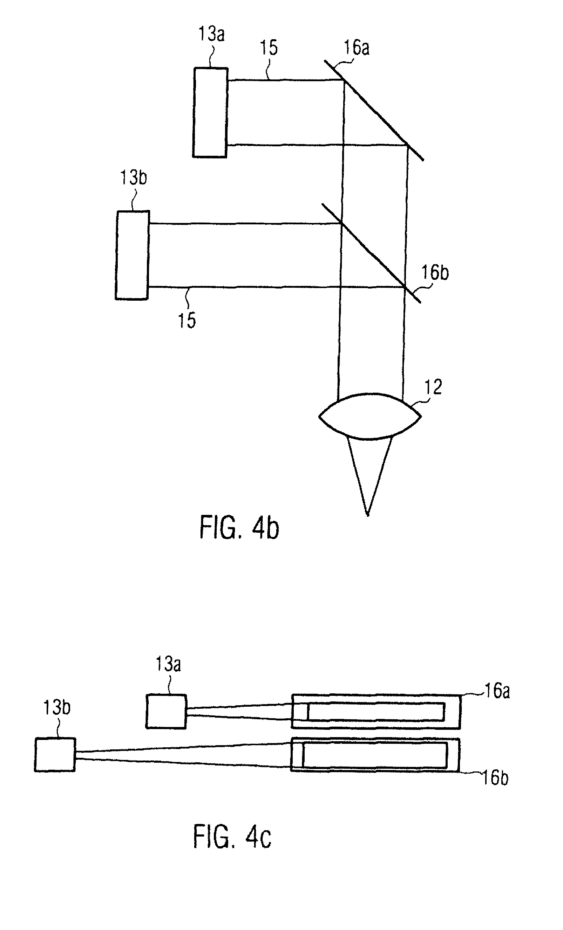

Fig. 4b und 4c zeigen Anordnungen in Drauf- und Vorderansicht, mit der das Licht aus verschiedenen Laserbarren gebündelt werden kann. Zwei Barren 13 a und 13 b sind höhenversetzt angeordnet. Sie geben das Licht 15 auf jeweils einen Spiegel 16a, 16b, die ebenfalls höhenversetzt angeordnet sind. Zwischen den Barren 13a, 13b und den Spiegeln 16a und 16b kann jeweils noch ein Zylinderlinsenarray angeordnet sein, um einen (wenigstens in etwa) kollimierten Strahl 15 zu erhalten. Die von den Spiegeln 16a, 16b reflektierten Strahlen laufen parallel und übereinander, so dass sie von einer einzelnen Einkoppeloptik 12 fokussiert werden können. Der optische Weg zwischen der Einkoppeloptik 12 und den Laserbarren 13a, 13b ist vorteilhafterweise jeweils in etwa gleich lang.

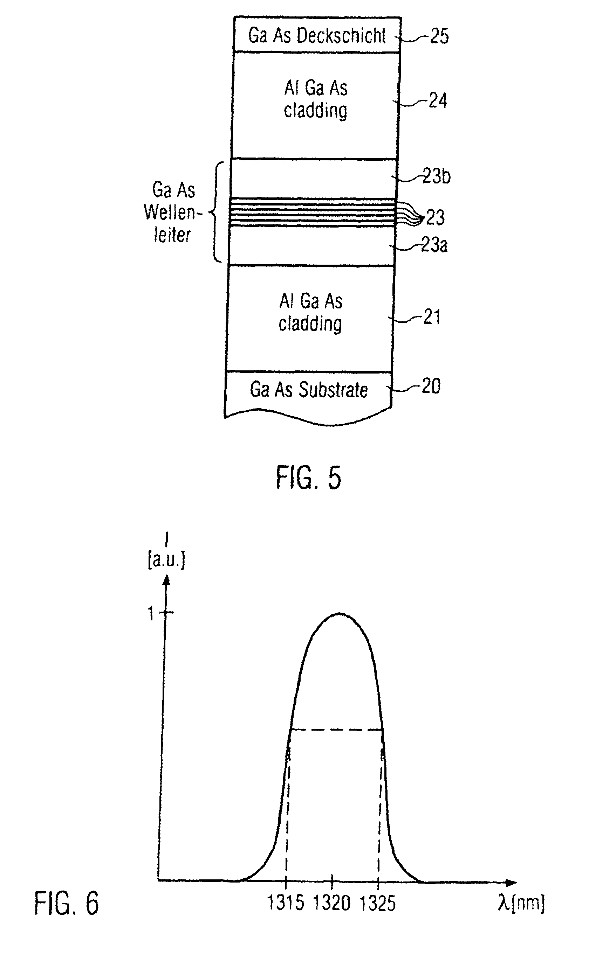

Eine schematische Darstellung der Schichtstruktur des Halbleiterlasers ist in Figur 5 dargestellt. Auf einem Substrat 20 wird eine Claddingschicht 21 aufgewachsen. Auf dieser wird der Wellenleiter ausgebildet. Der Wellenleiter hat einen Brechungsindex, der höher ist, als der der Claddingschicht. Dadurch führt der Wellenleiter die Lichtmode in dem Halbleitermaterial. Innerhalb des Wellenleiters sind verschiedene Schichten 23 angeordnet, die Quantenfilme sein können. In diesen Quantenfilmen 23 können dann auch noch Quantenpunkte angeordnet sein, die die Laserwellenlänge des Lasers festlegen.

Auch ist es möglich nur Quantenpunkte ohne Quantentilme vorzusehen.

Die Quantenpunkte können auch die Form von Quantendashes haben. Hierbei handelt es sich um Quantenpunkte, die eine länglich Form haben, wie etwa ein lang gezogenes Sechseck oder Ähnliches.

Auf dem Wellenleiter wird eine weitere Claddingschicht 24 aufgebracht und eventuell noch eine Deckschicht 25 angeordnet. Das Substrat 20 und die Claddingschicht 21 weisen vorzugsweise eine gleiche Dotierung auf. Die Claddingschicht 24 und die Deckschicht 25 weisen die entgegengesetzte Dotierung auf. Der Wellenleiter hat beispielsweise eine Breite von 500 nm und kann somit als LOC (Large Optical Cavity) bezeichnet werden. Die Claddingschichten können beispielsweise AlₓGa₁₋ₓAs umfassen bzw. daraus bestehen. Der Aluminiumgehalt und die Dicke dieser Schichten kann geeignet angepasst werden. Die Dicke kann beispielsweise 1,6 Mikrometer sein.

Allgemein können auch δ-dotierte Schichten (δ-doped layers) in dem aktiven Bereich vorgesehen sein, um hohe Laserleistungen zu erreichen.

Der Wellenleiter kann beispielsweise GaAs sein oder umfassen. Ihm kann auch Aluminium, Indium, Phosphor oder Ähnliches hinzugegeben werden. Seine Bandlücke ist kleiner als die der Claddingschicht 21, 24. Das Material des Wellenleiters 23 a, 23 b ist vorzugsweise undotiert.

Werden die Quantenpunkte beispielsweise aus lnGaAs gebildet, so können sie auch in In-GaAs-Quantenfilmen gebildet werden, sofern der Indiumgehalt in den Punkten wesentlich größer ist.

Die Quantenfilme 23 können auch ohne Quantenpunkte ausgebildet werden. Beispielsweise kann hier GalnAsN als Filmmaterial vorgesehen sein. Diese Materialschichten können zwischen GaAs und/oder GalnAs angeordnet werden. Sowohl die Quantenfilmmateriaien als auch die Barrieren dazwischen können mit Antimon (Sb) oder Phosphor angepasst werden. Dadurch kann die genauer gewünschte Wellenlänge eingestellt werden.

Statt GalnAsN kann auch GalnAsP als Quantenfilm oder Quantenpunktmaterial eingesetzt werden.

Figur 6 zeigt ein typisches Spektrum des durch die Laservorrichtung ausgegebenen Laserlichts. In Figur 6 ist die Intensität in willkürlichen Einheiten über der Wellenlänge in Nanometern aufgetragen. Das dort gezeigte Spektrum hat eine Halbwertsbreite von 10 Nanometern und eine Zentralwellenlänge von 1320 Nanometern.

## Patentansprüche

1. Vorrichtung für die Laserchirurgie mit:
einem Laser (13) und
Mitteln (12, 8) zum Einkoppeln von Laserlicht des Lasers (13) in eine Lichtleitfaser (11, 3) **dadurch gekennzeichnet, dass**
der Laser (13) ein Festkörperlaser ist, der Laserlicht (15) ausstrahlen kann, das bei der Maximalintensität eine Wellenlänge im Bereich zwischen 1100 und 1400 nm hat,
wobei der Laser mindestens eine Ausgangsleistung von 25 W hat.

2. Vorrichtung nach Anspruch 1, **dadurch gekennzeichnet, dass** der Wellenlängenbereich der Maximalintensität oberhalb von 1150, 1175, 1200, 1250, 1275, 1300, 1308 oder 1310 nm
und/oder unterhalb von 1370, 1350, 1330, 1325, 1300, 1275, 1250, 1225 oder 1200 nm liegt.

3. Vorrichtung nach einem der Ansprüche 1 oder 2, **dadurch gekennzeichnet, dass** die spektrale Halbwertsbreite des Laserlichts mindestens 2, 3, 4, 5, 7, 10, 12 oder 15 nm und/oder höchstens 2, 3, 4, 5, 7, 10, 12, 15, 20, 25, 30, 40 nm beträgt.

4. Vorrichtung nach einem der Ansprüche 1 bis 3, **dadurch gekennzeichnet, dass** die Ausgangsleistung des Lasers (13) mindestens 30, 35, 40, 45, 50, 60, 70, 80, 90 oder 100 W beträgt.

5. Vorrichtung nach einem der Ansprüche 1 bis 4, **dadurch gekennzeichnet, dass** der Laser (13) ein Dauerstrichlaser ist.

6. Vorrichtung nach einem der Ansprüche 1 bis 4, **dadurch gekennzeichnet, dass** der Laser (13) ein gepulster Laser (13) ist.

7. Vorrichtung nach einem der Ansprüche 1 bis 6, **dadurch gekennzeichnet, dass** der Laser (13) mehrere Laserelemente (14) umfasst, wie etwa mindestens oder genau 2, 5, 10, 15, 19, 20, 25, 30, 35, 38, 40, 50, 60, 70, 80, 90, 100, 120 oder 150 Laserelemente,
wobei vorzugsweise Mittel (12) zum Einkoppeln des Laserlichts der einzelnen Laserelemente (14) in eine gemeinsame Lichtleitfaser (10) vorgesehen sind.

8. Vorrichtung nach einem der Ansprüche 1 bis 7, **dadurch gekennzeichnet, dass** der Festkörperlaser ein Faserlaser ist oder umfasst.

9. Vorrichtung nach einem der Ansprüche 1 bis 8, **dadurch gekennzeichnet, dass** der Festkörperlaser ein Halbleiterlaser ist oder umfasst,
wobei der Wellenleiter (22) des Halbleiterlasers vorzugsweise eine Breite von mindestens 200, 250, 300, 400, 500 oder 600 nm hat,
und/oder wobei der Wellenleiter (22) vorzugsweise GaₓIn₍₁₋ₓ₎AsᵤPᵥN_{w}Sb_{(1-u-v-w)} umfasst oder daraus besteht, wobei x, u, v und w die Werte 0 bis 1 und alle Zwischenwerte oder Werte aus allen möglichen Zwischenintervallen annehmen kann und u+v+w kleiner oder gleich 1 ist.

10. Vorrichtung nach Anspruch 9, **dadurch gekennzeichnet, dass** der Laser (13) oder die Laserelemente (14) Quantenpunktlaser und/oder Quantenfilmlaser sind oder umfassen.

11. Vorrichtung nach Anspruch 10, **dadurch gekennzeichnet, dass** die Quantenpunktlaser GaAs und AlGaAs umfassen oder InP und AlGaAsP umfassen,
und/oder dass der/die Quantenpunktlaser Quantenpunkte in einem Quantenfilm (23) oder außerhalb von einem Quantenfilm oder in einem Wellenleitermaterial ohne Quantenfilm umfassen,
und/oder dass die Quantenpunkte GaₓIn₁₋ₓAs, GaₓIn₁₋ₓAs_{y}P_{1-y}, GaₓIn₁₋ₓAs_{y}N_{1-y} und/oder GaₓIn₁₋ₓSb umfassen oder daraus bestehen, wobei x und/oder y die Werte von 0 bis 1 annehmen kann.

12. Vorrichtung nach Anspruch 10, **dadurch gekennzeichnet, dass** der Quantenfilmlaser einen Quantenfilm (23) umfasst, der GaᵤIn₁₋ᵤAsₓN_{y}P_{(1-x-y)} umfasst oder daraus besteht, wobei y die Werte 0 bis 1 oder alle Zwischenwerte oder Werte aus allen möglichen Zwischenintervallen annehmen kann, solange x+y≤1 ist.

13. Vorrichtung nach einem der Ansprüche 9 bis 12, **dadurch gekennzeichnet, dass** der Festkörperlaser ein Scheibenlaser ist oder umfasst.

14. Vorrichtung nach einem der Ansprüche 1 bis 13, **dadurch gekennzeichnet, dass** eine Kopplung (8) zum lösbaren Anschließen einer Lichtleitfaser (3) vorgesehen ist

15. Vorrichtung nach einem der Ansprüche 1 bis 14, **dadurch gekennzeichnet, dass** die Vorrichtung eine lösbare Lichtleitfaser (3) umfasst,
wobei die Lichtleitfaser (3) vorzugsweise ein Griffteil (4) umfasst.

16. Vorrichtung nach Anspruch 15, **dadurch gekennzeichnet, dass** am distalen Ende der Lichtleitfaser (3) eine Optik (7) vorgesehen ist, mit der ein Lichtfokus in einem Abstand (d) von 1 bis 6 cm, bevorzugterweise 1 bis 5 cm, und noch bevorzugter 1,5 bis 3 cm, von dem Ende der Lichtleitfaser ausgebildet werden kann, wobei die Optik vorzugsweise auswechselbar ist,
und/oder wobei die Optik einen Fokus mit einer Halbwertsbreite (b) von 0,2 bis 0,7 mm, vorzugsweise 0,3 bis 0,6 mm und noch bevorzugter 0,4 bis 0,5 mm, erzeugt.

17. Vorrichtung nach einem der Ansprüche 1 bis 16, **dadurch gekennzeichnet, dass** eine Luftkühleinrichtung (5) zum Kühlen des Lasers mit Kühlluft vorgesehen ist.
